# EUROPEAN PATENT APPLICATION

(11) **EP 2 745 706 A1**
(43) Date of publication of application: **25.06.2014**
(21) Application number: 12382533.3
(22) Date of filing: 24.12.2012
(51) Int. Cl.: A23L 1/30, A23L 1/29, A61K 31/20

(54) **Methods for maintaining bone quality**

(71) Applicant: Abbott Laboratories, Inc., Abbott Park, IL 60064 (US)
(72) Inventor: BUENO VARGAS, María del Pilar, 18012 GRANADA (ES); LÓPEZ PEDROSA, José María, 18190 CENES DE LA VEGA (Granada) (ES); MANZANO MARTÍN, Manuel Cristóbal, 18151 GRANADA (ES); RUEDA CABRERA, Ricardo, 18008 GRANADA (ES)
(74) Representative: Ungria López, Javier

(57) **Abstract**

Methods for maintaining bone quality in an individual during pregnancy, during lactation, or during pregnancy and lactation are provided. The methods include providing an effective amount of a long chain polyunsaturated fatty acid, an effective amount of a prebiotic, or combinations thereof. The effective amount of the long chain polyunsaturated fatty acid, the effective amount of the prebiotic, or combinations thereof may be provided in a nutritional composition. The bone quality that is maintained may include bone microarchitecture, bone mineral density, bone mineral content, or combinations thereof.

## Description

### FIELD OF THE DISCLOSURE

The present disclosure relates to methods for maintaining bone quality in an individual during pregnancy, during lactation, or during pregnancy and lactation. More particularly, the present disclosure relates to the use of a long chain polyunsaturated fatty acid, a prebiotic, or both a long chain polyunsaturated fatty acid and a prebiotic to maintain bone quality in an individual during pregnancy, during lactation, or during pregnancy and lactation.

### BACKGROUND OF THE DISCLOSURE

During pregnancy and lactation there are significant alterations in maternal bone quality. In particular, during lactation there is a dramatic increase in the rate of maternal bone resorption, as well as an increase in calcium losses from the maternal skeleton (since the maternal skeleton serves as an important source of calcium for milk production). Accordingly, decreases in maternal bone mineral density are associated with pregnancy and lactation. Moreover, such decreases may increase the risk of bone fractures, the development of osteoporosis, or both later in the life of the mother.

### SUMMARY OF THE DISCLOSURE

Provided herein are methods for maintaining bone quality in an individual during pregnancy, during lactation, or during pregnancy and lactation. In one aspect of the general inventive concept, a long chain polyunsaturated fatty acid is used to maintain bone quality in a pregnant or lactating individual. In another aspect of the general inventive concept, a prebiotic is used to maintain bone quality in a pregnant or lactating individual. In certain embodiments, the bone quality that is maintained is bone microarchitecture, bone mineral density, bone mineral content, or combinations thereof. Use of the methods described herein may prevent or reduce the risk of bone fracture, prevent or reduce the development of osteoporosis, or both in the maternal individual later in life.

In a first embodiment, a method for maintaining bone quality in a pregnant or lactating individual is provided. The method includes providing an effective amount of a long chain polyunsaturated fatty acid for oral consumption by an individual during pregnancy, during lactation, or during pregnancy and lactation. Upon consumption of the effective amount of the long chain polyunsaturated fatty acid, the bone quality of the individual is maintained.

In a second embodiment, a method for maintaining bone quality in a pregnant or lactating individual is provided. The method includes providing an effective amount of a prebiotic for oral consumption by an individual during pregnancy, during lactation, or during pregnancy and lactation. Upon consumption of the effective amount of the prebiotic, the bone quality of the individual is maintained.

### BRIEF DESCRIPTION OF THE DRAWINGS

FIG. 1 depicts the effects of long chain polyunsaturated fatty acid (LCPUFA) supplementation on tibia trabecular thickness of rat dams as analyzed in Example 1;
FIG. 2 depicts the effects of LCPUFA supplementation on tibia trabecular bone surface-bone volume ratio (BS/BV) of rat dams as analyzed in Example 1;
FIG. 3 depicts the effects of LCPUFA supplementation on tibia Structure Model Index (SMI) of rat dams as analyzed in Example 1;
FIG. 4 depicts the effects of prebiotic supplementation on tibia trabecular thickness of rat dams as analyzed in Example 2;
FIG. 5 depicts the effects of prebiotic supplementation on tibia bone volume to total volume ratio (BV/TV) of rat dams as analyzed in Example 2;
FIG. 6 depicts the effects of prebiotic supplementation on tibia connective density of rat dams as analyzed in Example 2;
FIG. 7 depicts the effects of prebiotic supplementation on tibia trabecular bone surface-bone volume ratio (BS/BV) of rat dams as analyzed in Example 2; and
FIG. 8 depicts the effects of prebiotic supplementation on tibia Structure Model Index (SMI) of rat dams as analyzed in Example 2.

### DETAILED DESCRIPTION OF THE DISCLOSURE

Provided herein are methods for maintaining bone quality in an individual during pregnancy, during lactation, or during pregnancy and lactation. The methods include providing an effective amount of a long chain polyunsaturated fatty acid (LCPUFA), an effective amount of a prebiotic, or both to a pregnant or lactating individual.

The terminology as set forth herein is for description of the embodiments only and should not be construed as limiting the disclosure as a whole. Unless otherwise specified, "a," "an," "the," and "at least one" are used interchangeably. Furthermore, as used in the description and the appended claims, the singular forms "a," "an," and "the" are inclusive of their plural forms, unless the context clearly indicates otherwise.

The term "nutritional composition" as used herein, unless otherwise specified, refers to nutritional products in various forms including, but not limited to, liquids, solids, powders, semi-solids, semi-liquids, nutritional supplements, and any other nutritional food product known in the art. A nutritional composition in powder form may be reconstituted (upon addition of water or another liquid) to form a nutritional composition in liquid form. In certain embodiments according to the first and second embodiments, the nutritional compositions disclosed herein comprise at least one source of protein, at least one source of carbohydrate, and at least one source of fat. The nutritional compositions disclosed herein are generally suitable for oral consumption by a human.

The term "nutritional liquid" as used herein, unless otherwise specified, refers to nutritional compositions in ready-to-drink liquid form, concentrated liquid form, and nutritional liquids made by reconstituting nutritional powders described herein prior to use. The nutritional liquid may also be formulated as a suspension, an emulsion, a solution, and so forth.

The term "nutritional powder" or "reconstitutable powder" as used herein, unless otherwise specified, refers to nutritional compositions in flowable or scoopable form that can be reconstituted with water or another aqueous liquid prior to consumption and includes both spray dried and drymixed/dryblended powders.

The term "nutritional semi-solid" as used herein, unless otherwise specified, refers to nutritional products that are intermediate in properties, such as rigidity, between solids and liquids. Some semi-solids examples include puddings, yogurts, gels, gelatins, and doughs.

The term "nutritional semi-liquid" as used herein, unless otherwise specified, refers to nutritional compositions that are intermediate in properties, such as flow properties, between liquids and solids. Some semi-liquids examples include thick shakes, liquid yogurts, and liquid gels.

The term "serving" as used herein, unless otherwise specified, is intended to be construed as any amount which is intended to be consumed by an individual in one sitting or within one hour or less.

The term "bone quality" as used herein, unless otherwise specified, refers to characteristics that provide an indication of bone strength. For example, bone quality includes characteristics such as bone microarchitecture, bone mineral density (BMD), bone mineral content (BMC), bone morphology, accumulated microscopic damage, bone turnover, and so forth.

The phrase "effective amount," as used herein, refers to a sufficient amount of an ingredient (e.g., LCPUFA or prebiotic) to maintain bone quality or increase bone quality in the individual. The exact amount required will vary from individual to individual, depending on the species, age, weight, lifestyle and general condition of the particular individual.

In a first embodiment, a method for enhancing bone quality in a pregnant or lactating individual is provided. The method comprises providing an effective amount of a LCPUFA for oral consumption by an individual during pregnancy, during lactation, or during pregnancy and lactation. Upon consumption of the effective amount of the LCPUFA, the bone quality of the individual is maintained. As described further herein, more than one LCPUFA may be utilized in certain embodiments according to the methods of the first embodiment.

In a second embodiment, a method for maintaining bone quality in a pregnant or lactating individual is provided. The method includes providing an effective amount of a prebiotic for oral consumption by an individual during pregnancy, during lactation, or during pregnancy and lactation. Upon consumption of the effective amount of the prebiotic, the bone quality of the individual is maintained. As described further herein, more than one prebiotic may be utilized in certain embodiments according to the methods of the second embodiment.

According to the first embodiment, an effective amount of LCPUFA is provided to a pregnant or lactating individual for oral consumption. In certain embodiments according to the first embodiment, the effective amount of LCPUFA is provided to an individual during pregnancy, and subsequently to the individual during lactation/breastfeeding of the offspring. In other embodiments according to the first embodiment, the effective amount of LCPUFA is provided to an individual during either pregnancy or during lactation. Generally, the effective amount of LCPUFA may be provided in any form suitable for oral consumption by an individual. For example, the LCPUFA may be provided as caplets, tablets, pills, capsules, chewable tablets, quick dissolve tablets, effervescent tablets, solutions, suspensions, emulsions, multi-layer tablets, bi-layer tablets, soft gelatin capsules, hard gelatin capsules, lozenges, chewable lozenges, beads, granules, particles, microparticles, dispersible granules, cachets, and combinations thereof. In certain embodiments according to the first embodiment, the LCPUFA is provided as part of a nutritional composition, which will be discussed in more detail below.

In general, the LCPUFA used in the methods of the first embodiment has 18 or more carbons in its carbon chain. In certain embodiments, the LCPUFA is selected from the group consisting of n-3 fatty acids, n-6 fatty acids, and combinations thereof. Examples of LCPUFAs that may be utilized in the methods described herein include docosahexaenoic acid (DHA), eicosapentaenoic acid (EPA), arachidonic acid (ARA), linoleic acid, linolenic acid (including α-linolenic acid, or γ-linolenic acid, or combinations thereof), and combinations thereof. In certain embodiments according to the first embodiment, the LCPUFA is DHA. In certain other embodiments of the first embodiment, the LCPUFA is EPA. In one exemplary embodiment according to the first embodiment, the LCPUFA comprises DHA and EPA. Thus, in certain embodiments according to the first embodiment, the LCPUFA comprises a single LCPUFA, two LCPUFAs, three LCPUFAs, and so forth. The LCPUFAs may be derived from a variety of sources including, but not limited to, plant oils, marine phytoplankton, fungal oils, fish oils, and combinations thereof. One commercially available LCPUFA source is Eupoly-DHA™ (Puleva Biotech, Spain), which is a refined marine oil including about 10% EPA and about 20% DHA.

In certain embodiments according to the first embodiment, the effective amount of LCPUFA is consumed daily by the individual during pregnancy. In certain embodiments where the LCPUFA is consumed by the individual during pregnancy, the consumption takes place every day for: at least 1 month, at least 2 months, at least 3 months, at least 4 months, at least 5 months, at least 6 months, at least 7 months, at least 8 months, at least 9 months during the pregnancy or in some instances daily during the entire period of pregnancy. Taking into account that some individuals will not recognize that they are pregnant on day 1 of their pregnancy, it should be understood that the methods of the first embodiment are intended to encompass consumption of the LCPUFA (or the nutritional composition containing LCPUFA) on less than every day of the period of pregnancy. In other embodiments according to the first embodiment, the effective amount of LCPUFA is consumed by the individual on at least 1 day per week, at least 2 days per week, at least 3 days per week, at least 4 days per week, at least 5 days per week or 6 days per week during pregnancy. This periodic consumption may take place for: at least 1 month, at least 2 months, at least 3 months, at least 4 months, at least 5 months, at least 6 months, at least 7 months, at least 8 months, at least 9 months during the pregnancy or in some instances during the entire period of pregnancy. (For all embodiments described herein relating to administering a LCPUFA to an individual (or an individual consuming a LCPUFA), each day is intended to reflect that an individual has been instructed to consume the LCPUFA (or the nutritional composition containing the LCPUFA) each day, and that the individual is actually administered the LCPUFA (or the nutritional composition containing the LCPUFA) for at least 90% of the days during the desired period of time.)

In certain embodiments according to the first embodiment, the effective amount of LCPUFA is consumed daily by the individual during lactation (*i.e.,* while breastfeeding the offspring). In certain embodiments where the LCPUFA is consumed by the individual during lactation, the consumption takes place every day for: at least 1 month, at least 2 months, at least 3 months, at least 4 months, at least 5 months, at least 6 months, at least 9 months, at least 12 months, at least 18 months, and up to 24 months from delivery of the offspring. It should be understood that the methods of the first embodiment are intended to encompass consumption of the LCPUFA (or a nutritional composition containing LCPUFA) on less than every day of the period of lactation. In other embodiments according to the first embodiment, the effective amount of LCPUFA is consumed by the individual on at least 1 day per week, at least 2 days per week, at least 3 days per week, at least 4 days per week, at least 5 days per week or 6 days per week during lactation. This periodic consumption may take place for: at least 1 month, at least 2 months, at least 3 months, at least 4 months, at least 5 months, at least 6 months, at least 9 months, at least 12 months, at least 18 months, and up to 24 months or during the entire period of lactation (*i.e.*, the entire period of time when the individual breastfeeds the offspring).

In certain embodiments according to the first embodiment, the effective amount of LCPUFA is consumed daily by the individual during both pregnancy and lactation (*i.e*., while breastfeeding the offspring). In certain embodiments where the LCPUFA is consumed by the individual during both pregnancy and lactation, the consumption takes place every day for: at least 1 month, at least 2 months, at least 3 months, at least 4 months, at least 5 months, at least 6 months, at least 7 months, at least 8 months, at least 9 months during the pregnancy or in some instances daily during the entire period of pregnancy. Taking into account that some individuals will not recognize that they are pregnant on day 1 of their pregnancy, it should be understood that the methods of the first embodiment are intended to encompass consumption of the LCPUFA (or a nutritional composition containing LCPUFA) on less than every day of the period of pregnancy and on less than every day of the period of lactation. In other embodiments according to the first embodiment, the effective amount of LCPUFA is consumed by the individual on at least 1 day per week, at least 2 days per week, at least 3 days per week, at least 4 days per week, at least 5 days per week or 6 days per week during pregnancy and during lactation. This periodic consumption may take place for: at least 1 month, at least 2 months, at least 3 months, at least 4 months, at least 5 months, at least 6 months, at least 7 months, at least 8 months, at least 9 months during the pregnancy or in some instances during the entire period of pregnancy. Similarly, the periodic consumption described may take place during similar periods or during the entire period of lactation. It should be understood that the schedule of administration or consumption during the period of pregnancy may be the same or different than the schedule of administration or consumption during the period of lactation.

In certain embodiments of the first embodiment, the effective amount of LCPUFA consumed (during pregnancy, during lactation, or during pregnancy and lactation) ranges from 100 mg/day to 3000 mg/day, including from 200 mg/day to 2000 mg/day, including from 500 mg/day to 1500 mg/day, and also including from 1000 mg/day to 1250 mg/day. It should be understood that in those embodiments where a LCPUFA is consumed both during pregnancy and during lactation, that the effective amount of LCPUFA consumed during each period may be the same or different. In certain embodiments according to the first embodiment, the effective amount of LCPUFA is provided in one to four doses, or servings, per day. In certain embodiments according to the first embodiment, the effective amount of LCPUFA is provided in two servings per day.

In certain embodiments of the first embodiment, the effective amount of LCPUFA consumed (during pregnancy, during lactation, or during pregnancy and lactation) ranges from 25 mg/serving to 1 g/serving, including from 50 mg/serving to 750 mg/serving, including from 125 mg/serving to 500 mg/serving, and also including from 250 mg/serving to 325 mg/serving. It should be understood that in those embodiments where a LCPUFA is consumed both during pregnancy and during lactation, that the effective amount of LCPUFA consumed during each period may be the same or different. In certain embodiments of the first embodiment, when the effective amount of LCPUFA is provided as a liquid, the serving is 8 fl oz to 14 fl oz, including 8 fl oz to 12 fl oz, and also including 8 fl oz to 10 fl oz.

According to the second embodiment, an effective amount of a prebiotic is provided to a pregnant or lactating individual for oral consumption. In certain embodiments according to the second embodiment, the effective amount of the prebiotic is provided to an individual during pregnancy, and subsequently to the individual during lactation and breastfeeding of the offspring. In other embodiments according to the second embodiment, the effective amount of prebiotic is provided to an individual during either pregnancy or during lactation. Generally, the effective amount of prebiotic may be provided in any form suitable for oral consumption by an individual. For example, the prebiotic may be provided as caplets, tablets, pills, capsules, chewable tablets, quick dissolve tablets, effervescent tablets, solutions, suspensions, emulsions, multi-layer tablets, bi-layer tablets, soft gelatin capsules, hard gelatin capsules, lozenges, chewable lozenges, beads, granules, particles, microparticles, dispersible granules, cachets, and combinations thereof. In certain embodiments according to the second embodiment, the prebiotic is provided as part of a nutritional composition, which will be discussed in more detail below.

The term "prebiotic" as used herein refers to a non-digestible food ingredient that selectively stimulates the growth or activity of certain gastrointestinal microbiota. Examples of prebiotics that may be utilized in the methods described herein include inulin, short and long chain fructooligosaccharides (FOS), galactooligosaccharides (GOS), xylooligosaccharides (XOS), galactofructose, isomaltooligosaccharides (IMO), polydextrose, lactosucrose, acacia gum, resistant starch, dextrans, and combinations thereof. In certain embodiments according to the second embodiment, the prebiotic is FOS, or inulin, or combinations thereof. Thus, in certain embodiments according to the second embodiment, the prebiotic comprises a single prebiotic ingredient, two prebiotic ingredients, three prebiotic ingredients, and so forth. The prebiotics may be derived from various plant sources (e.g., soybean, chicory root, Jerusalem artichoke, etc.) or may be derived synthetically.

In certain embodiments according to the second embodiment, the effective amount of the prebiotic is consumed daily by the individual during pregnancy. In certain embodiments where the prebiotic is consumed by the individual during pregnancy, the consumption takes place every day for: at least 1 month, at least 2 months, at least 3 months, at least 4 months, at least 5 months, at least 6 months, at least 7 months, at least 8 months, at least 9 months during the pregnancy or in some instances daily during the entire period of pregnancy. Taking into account that some individuals will not recognize that they are pregnant on day 1 of their pregnancy, it should be understood that the methods of the second embodiment are intended to encompass consumption of the prebiotic (or a nutritional composition containing prebiotic) on less than every day of the period of pregnancy. In other embodiments according to the second embodiment, the effective amount of prebiotic is consumed by the individual on at least 1 day per week, at least 2 days per week, at least 3 days per week, at least 4 days per week, at least 5 days per week, or 6 days per week during pregnancy. This periodic consumption may take place for: at least 1 month, at least 2 months, at least 3 months, at least 4 months, at least 5 months, at least 6 months, at least 7 months, at least 8 months, at least 9 months during the pregnancy or in some instances during the entire period of pregnancy. (For all embodiments described herein relating to administering a prebiotic to an individual (or an individual consuming a prebiotic), each day is intended to reflect that an individual has been instructed to consume the prebiotic (or a nutritional composition containing the prebiotic) each day, and that the individual is actually administered the prebiotic (or a nutritional composition containing the prebiotic) for at least 90% of the days during the desired period of time.)

In certain embodiments according to the second embodiment, the effective amount of the prebiotic is consumed daily by the individual during lactation (i.e., while breastfeeding the offspring). In certain embodiments where the prebiotic is consumed by the individual during lactation, the consumption takes place every day for: at least 1 month, at least 2 months, at least 3 months, at least 4 months, at least 5 months, at least 6 months, at least 9 months, at least 12 months, at least 18 months, and up to 24 months from delivery of the offspring. It should be understood that the methods of the second embodiment are intended to encompass consumption of the prebiotic (or a nutritional composition containing prebiotic) on less than every day of the period of lactation. In other embodiments according to the second embodiment, the effective amount of the prebiotic is consumed by the individual on at least 1 day per week, at least 2 days per week, at least 3 days per week, at least 4 days per week, at least 5 days per week or 6 days per week during lactation. This periodic consumption may take place for: at least 1 month, at least 2 months, at least 3 months, at least 4 months, at least 5 months, at least 6 months, at least 9 months, at least 12 months, at least 18 months, and up to 24 months or during the entire period of lactation (i.e., the entire period of time when the individual breastfeeds the offspring).

In certain embodiments according to the second embodiment, the effective amount of the prebiotic is consumed daily by the individual during both pregnancy and lactation (i.e., while breastfeeding the offspring). In certain embodiments where the prebiotic is consumed by the individual during both pregnancy and lactation, the consumption takes place every day for: at least 1 month, at least 2 months, at least 3 months, at least 4 months, at least 5 months, at least 6 months, at least 7 months, at least 8 months, at least 9 months during the pregnancy or in some instances daily during the entire period of pregnancy. Taking into account that some individuals will not recognize that they are pregnant on day 1 of their pregnancy, it should be understood that the methods of the second embodiment are intended to encompass consumption of the prebiotic (or a nutritional composition containing prebiotic) on less than every day of the period of pregnancy and on less than every day of the period of lactation. In other embodiments according to the second embodiment, the effective amount of prebiotic is consumed by the individual on at least 1 day per week, at least 2 days per week, at least 3 days per week, at least 4 days per week, at least 5 days per week, or 6 days per week during pregnancy and during lactation. This periodic consumption may take place for: at least 1 month, at least 2 months, at least 3 months, at least 4 months, at least 5 months, at least 6 months, at least 7 months, at least 8 months, at least 9 months during the pregnancy or in some instances during the entire period of pregnancy. Similarly, the periodic consumption described may take place during similar periods or during the entire period of lactation. It should be understood that the schedule of administration or consumption during the period of pregnancy may be the same or different than the schedule of administration or consumption during the period of lactation.

In certain embodiments of the second embodiment, the effective amount of prebiotic consumed (during pregnancy, during lactation, or during pregnancy and lactation) ranges from 0.001 g/day to 15 g/day, including from 0.001 g/day to 10 g/day, and including from 0.1 g/day to about 5 g/day. It should be understood that in those embodiments where a prebiotic is consumed both during pregnancy and during lactation, that the effective amount of prebiotic consumed during each period may be the same or different. In certain embodiments according to the second embodiment, the effective amount of prebiotic is provided in one to four doses, or servings, per day. In certain embodiments according to the second embodiment, the effective amount of prebiotic is provided in two servings per day. [0001] In certain embodiments of the second embodiment, the effective amount of prebiotic consumed (during pregnancy, during lactation, or during pregnancy and lactation) ranges from 500 mcg/serving to 7.5 g/serving, including from 500 mcg/serving to 5 g/serving, including from 50 mg/serving to 2.5 g/serving, and also including from 500 mg/serving to 1.25 g/serving. It should be understood that in those embodiments where the prebiotic is consumed both during pregnancy and during lactation, that the effective amount of prebiotic consumed during each period may be the same or different. In certain embodiments of the second embodiment, when the effective amount of prebiotic is provided as a liquid, the serving is 8 fl oz to 14 fl oz, including 8 fl oz to 12 fl oz, and also including 8 fl oz to 10 fl oz.

As mentioned above, in certain embodiments according to the first and second embodiments, the effective amount of LCPUFA and the effective amount of prebiotic is provided as part of a nutritional composition. In certain embodiments according to the first and second embodiments, the nutritional compositions are formulated as, and intended for consumption in, any known or otherwise suitable oral product form. Any solid, liquid, semi-solid, semi-liquid, or powder product form, including combinations or variations thereof, are suitable for use herein, provided that such forms allow for safe and effective oral delivery to the individual via oral consumption of the ingredients as also defined herein.

In certain embodiments of the first and second embodiments, the nutritional composition is a solid nutritional product. Non-limiting examples of solid nutritional products include snack and meal replacement products, including those formulated as bars, sticks, cookies or breads or cakes or other baked goods, frozen liquids, candy, breakfast cereals, powders or granulated solids or other particulates, snack chips or bites, frozen or retorted entrees and so forth. In certain embodiments according to the first and second embodiments, when the nutritional composition is a solid product, the serving is within a range of 25 grams to 150 grams. In certain embodiments according to the first and second embodiments, when the nutritional composition is a solid product, the individual is provided one to four servings per day.

In certain embodiments of the first and second embodiments, the nutritional composition is a nutritional liquid. Non-limiting examples of nutritional liquids include snack and meal replacement products, hot or cold beverages, carbonated or non carbonated beverages, juices or other acidified beverages, milk or soy-based beverages, shakes, coffees, teas, and so forth. Generally, the nutritional liquids are formulated as suspensions or emulsions, but the nutritional liquids can also be formulated in any other suitable forms such as clear liquids, solutions, liquid gels, liquid yogurts, and so forth.

In certain embodiments according to the first and second embodiments, when the nutritional composition is a liquid, the serving is within a range of 30 milliliters to 500 milliliters (~1 fl oz to ~17 fl oz). In certain other embodiments according to the first and second embodiments, when the nutritional composition is a liquid, the serving is 237 milliliters (~8 fl oz). In other embodiments according to the first and second embodiments, when the nutritional composition is a liquid, the serving is 177 milliliters to 417 milliliters (~6 fl oz to ~14 fl oz). In yet other embodiments according to the first and second embodiments, when the nutritional composition is a liquid, the serving is 207 milliliters to 296 milliliters (~7 fl oz to ~10 fl oz). In still other embodiments according to the first and second embodiments, when the nutritional composition is a liquid, the serving is 30 milliliters to 75 milliliters (~1 fl oz to ~ 2.5 fl oz). In certain embodiments according to the first and second embodiment, when the nutritional composition is provided as a liquid, 1 to 4 servings of the nutritional composition is provided to the individual each day.

In yet other embodiments according to the first and second embodiments, the nutritional composition may be formulated as semi-solid or semi-liquid compositions (*e.g.,* puddings, gels, yogurts, *etc*.), as well as more conventional product forms such as capsules, tablets, caplets, pills, and so forth. In other embodiments of the first and second embodiments, the nutritional composition may be in the form of lozenges, tablets (*e.g*., chewable, coated, *etc*.), pastes, gels, or yogurts.

The nutritional compositions disclosed herein are generally useful to provide sole, primary, or supplemental sources of nutrition, as well as providing one or more of the benefits as described herein. In certain embodiments according to the first and second embodiments, the nutritional composition provides up to 500 kcal of energy per serving or dose, including from 20 kcal to 500 kcal, from 75 kcal to 500 kcal, from 150 kcal to 500 kcal, from 150 kcal to 500 kcal, from 300 kcal to 500 kcal, or from 400 kcal to 500 kcal per serving or dose.

In certain embodiments according to the first embodiment, where the effective amount of LCPUFA is provided as part of a nutritional composition, the nutritional composition includes at least one source of protein, at least one source of carbohydrate, and optionally at least one source of fat in addition to the source of LCPUFA. In certain embodiments according to the first embodiment, where the effective amount of LCPUFA is provided as part of a nutritional composition, the nutritional composition may further comprise an effective amount of a prebiotic. In certain embodiments according to the first embodiment, where the effective amount of LCPUFA is provided as part of a nutritional composition, the nutritional composition comprises from 0.001% to 1% of LCPUFA by weight of the nutritional composition, including from 0.001% to 0.1%, including from 0.02% to 0.09%, including from 0.025% to 0.06%, and also including from 0.025% to 0.05% of LCPUFA by weight of the nutritional composition. In certain other embodiments according to the first embodiment, where the effective amount of LCPUFA is provided as part of a nutritional composition, the nutritional composition comprises from 0.01% to 1% of LCPUFA by weight of the nutritional composition, including from 0.1 % to 1%, including from 0.2% to 1%, including from 0.4% to 1%, including from 0.6% to 1%, and also including from 0.8% to 1% LCPUFA by weight of the nutritional composition.

In certain embodiments according to the second embodiment, where the effective amount of prebiotic is provided as part of a nutritional composition, the nutritional composition includes at least one source of protein, at least one source of carbohydrate, and at least one source of fat. In certain embodiments according to the second embodiment, where the effective amount of prebiotic is provided as part of a nutritional composition, the nutritional composition may further comprise an effective amount of LCPUFA. In certain embodiments according to the second embodiment, where the effective amount of prebiotic is provided as part of a nutritional composition, the nutritional composition comprises from 1% to 20% prebiotic by weight of the nutritional composition, including from 1% to 15%, including from 5% to 12%, and also including from 7% to 10% prebiotic by weight of the nutritional composition.

In certain embodiments according to the first and second embodiments, the nutritional composition comprises at least one source of protein in an amount sufficient to provide 6 grams to 50 grams of protein per serving of the nutritional composition. In certain embodiments according to the first and second embodiments, the nutritional composition comprises 6 grams to 50 grams of protein per serving, including 9 grams to 40 grams of protein, including 9 grams to 35 grams of protein, and also including 9 grams to 30 grams of protein per serving. In certain other embodiments according to the first and second embodiments, the at least one source of protein comprises 5% to 40% of the nutritional composition, by weight, including from 10% to 30%, and also including 15% to 25% by weight of the composition. Virtually any source of protein may be used so long as it is suitable for use in oral nutritional compositions and is otherwise compatible with any other selected ingredients or features in the nutritional composition.

The source of protein may include, but is not limited to, intact, hydrolyzed, and partially hydrolyzed protein, which may be derived from any known or otherwise suitable source such as milk (e.g., casein, whey), animal (e.g., meat, fish), cereal (e.g., rice, corn), vegetable (e.g., soy, potato, pea), and combinations thereof. Non-limiting examples of the source of protein include whey protein concentrates, whey protein isolates, whey protein hydrolysates, acid caseins, sodium caseinates, calcium caseinates, potassium caseinates, casein hydrolysates, milk protein concentrates, milk protein isolates, milk protein hydrolysates, nonfat dry milk, condensed skim milk, soy protein concentrates, soy protein isolates, soy protein hydrolysates, pea protein concentrates, pea protein isolates, pea protein hydrolysates, collagen proteins, and combinations thereof. In addition, the at least one source of protein in an amount sufficient to provide 6 grams to 50 grams of protein per serving may comprise any one source of protein or any combination of any of the various sources of protein provided in the non-limiting list presented above.

As mentioned, in certain embodiments according to the first and second embodiments, the nutritional composition further comprises at least one source of carbohydrate. In some embodiments according to the first and second embodiments, the at least one source of carbohydrate comprises from 10% to 80% of the nutritional composition, by weight, including from 30% to 60%, and also including from 50% to 70% by weight of the nutritional composition. In other embodiments according to the first and second embodiments, the nutritional composition comprises 15 grams to 110 grams of at least one source of carbohydrate per serving. In other embodiments according to the first and second embodiments, the nutritional composition comprises 25 grams to 90 grams of at least one source of carbohydrate per serving, including 40 grams to 65 grams of at least one source of carbohydrate per serving, and also including 45 grams to 55 grams of at least one source of carbohydrate per serving.

The at least one source of carbohydrate suitable for use in certain embodiments of the nutritional compositions disclosed herein may be simple, complex, or variations or combinations thereof. Generally, any source of carbohydrate may be used so long as it is suitable for use in oral nutritional compositions and is otherwise compatible with any other selected ingredients or features present in the nutritional composition. Non-limiting examples of a source of carbohydrate suitable for use in the nutritional compositions described herein include maltodextrin, hydrolyzed or modified starch or cornstarch, glucose polymers, corn syrup, corn syrup solids, rice-derived carbohydrates, sucrose, glucose, fructose, lactose, high fructose corn syrup, honey, sugar alcohols (e.g., maltitol, erythritol, sorbitol, etc.), and combinations thereof.

In addition, in certain embodiments according to the first and second embodiments, the nutritional composition further comprises at least one source of fat. In other embodiments according to the second embodiment, the nutritional composition comprises no fat, or essentially no fat (i.e., less than 0.5 grams of fat per serving). In some embodiments according to the first and second embodiments where the nutritional composition contains fat, the nutritional composition comprises from 2 grams to 45 grams of at least one source of fat per serving. In other embodiments according to the first and second embodiments, the nutritional composition comprises from 5 grams to 35 grams of at least one source of fat per serving, including from 10 grams to 30 grams of at least one source of fat per serving, and also including from 15 grams to 25 grams of at least one source of fat per serving. In certain embodiments according to the first and second embodiments where the nutritional composition comprises at least one source of fat, the at least one source of fat comprises from 5% to 30% of the nutritional composition, by weight, including from 10% to 25% by weight of the nutritional composition, and also including from 12% to 18% by weight of the nutritional composition.

In general, any source of fat may be used so long as it is suitable for use in oral nutritional compositions and is otherwise compatible with any other selected ingredients or features present in the nutritional composition. The source of fat may be derived from plants, animals, and combinations thereof. Non-limiting examples of suitable sources of fat for use in the nutritional compositions described herein include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, and combinations thereof.

In accordance with certain embodiments of the second embodiment, the nutritional composition is formulated as a clear liquid having a pH of 2 to 5, and also having no more than 0.5% fat by weight of the nutritional composition. The limited amount of fat contributes to the desired clarity and the desired pH of the nutritional composition. Typically, liquid nutritional compositions desired to be clear, or at least substantially translucent, are substantially free of fat. As used herein "substantially free of fat" refers to nutritional compositions containing less than 0.5%, and including less than 0.1% fat by weight of the total composition. "Substantially free of fat" also may refer to nutritional compositions disclosed herein that contain no fat, i.e., zero fat. Furthermore, embodiments of liquid nutritional compositions that have a desired acidic pH in the range of 2 to 5 (e.g., juices, fruit juices, fruit-flavored beverages, etc.) are typically substantially free of fat. Liquid nutritional compositions that are both clear and have a pH ranging from 2 to 5 are also typically substantially free of fat. In certain of the preceding embodiments, the pH of the nutritional composition may be from 2.5 to 4.6, including a pH of 3 to 3.5. In those embodiments of the nutritional compositions that are substantially free of fat but have some amount of fat present, the fat may be present as a result of being inherently present in another ingredient (e.g., a source of protein) or may be present as a result of being added as one or more separate sources of fat.

In certain embodiments according to the first and second embodiments, the nutritional composition is formulated as an aqueous emulsion. The emulsion generally comprises at least one source of protein, at least one source of carbohydrate, and at least one source of fat. The emulsions are flowable or drinkable liquids at from about 1 to about 25 °C and are typically in the form of oil-in-water, water-in-oil, or complex aqueous emulsions, although such emulsions are most typically in the form of oil-in-water emulsions having a continuous aqueous phase and a discontinuous oil phase. The emulsion may have a pH ranging from 3.0 to 8, but is generally formulated with a pH in a range of from 5 to 8, including from 6 to 8, and also including from 6.5 to 7.5.

In certain embodiments according to the first and second embodiments disclosed herein, the nutritional composition may further comprise other optional components or ingredients that may modify the physical, chemical, aesthetic or processing characteristics of the nutritional composition or serve as pharmaceutical or additional nutritional components. Many such optional ingredients are known or otherwise suitable for use in medical food or other nutritional products or pharmaceutical dosage forms and may also be used in the nutritional compositions disclosed herein, provided that such optional ingredients are safe for oral administration and are compatible with the essential and other ingredients in the selected product form.

Non-limiting examples of such optional ingredients include preservatives, emulsifying agents, buffers, creatine, probiotics, pharmaceutical actives, antiinflammatory agents, additional nutrients, colorants, flavors, thickening agents and stabilizers, emulsifying agents, lubricants, and so forth.

In certain embodiments according to the first and second embodiments disclosed herein, the nutritional composition may further comprise at least one sweetening agent. In certain embodiments, the at least one sweetening agent is at least one sugar alcohol such as maltitol, erythritol, sorbitol, xylitol, mannitol, isolmalt, and lactitol, or at least one artificial or high potency sweetener such as acesulfame K, aspartame, sucralose, saccharin, stevia, and tagatose, and combinations thereof. The sweetening agents, especially as a combination of a sugar alcohol and an artificial sweetener, are especially useful in formulating liquid nutritional compositions having a desirable favor profile. These sweetener combinations are especially effective in masking undesirable flavors, for example, as sometimes associated with the addition of vegetable proteins to a liquid nutritional composition. In certain embodiments according to the first and second embodiments disclosed herein, the nutritional composition may comprise at least one sugar alcohol with a concentration in a range from at least 0.01%, including from about 0.1 % to about 10%, and also including from about 1% to about 6%, by weight of the nutritional composition. In certain embodiments according to the first and second embodiments disclosed herein, the nutritional composition may comprise at least one artificial sweetener with a concentration in a range from 0.01% to 5%, including from 0.05% to 3%, and also including from 0.1% to 1%, by weight of the nutritional composition.

A flowing agent or anti-caking agent may be included in certain embodiments of the nutritional composition according to the first and second embodiments disclosed herein to retard clumping or caking of a nutritional powder embodiment over time and to make the nutritional powder flow easily from its container. Any known flowing or anti-caking agents that are known or otherwise suitable for use in a nutritional powder or product form are suitable for use herein, non-limiting examples of which include tricalcium phosphate, silicates, and combinations thereof. The concentration of the flowing agent or anti-caking agent in certain embodiments of the nutritional composition according to the first and second embodiments disclosed herein varies depending upon the product form, the other selected ingredients, the desired flow properties, and so forth, but most typically range from about 0.1% to about 4%, including from about 0.5% to about 2%, by weight of the nutritional composition.

In certain embodiments according to the first and second embodiments disclosed herein, the nutritional composition may comprise a stabilizer. Any stabilizer that is known or otherwise suitable for use in a nutritional composition is also suitable for use herein, some non-limiting examples of which include gums such as xanthan gum. In certain embodiments according to the first and second embodiments disclosed herein, the stabilizer may represent from about 0.1% to about 5%, including from about 0.5% to about 3%, including from about 0.7% to about 1.5%, by weight of the nutritional composition.

In certain other embodiments according to the first and second embodiments disclosed herein, the nutritional composition may further comprise any of a variety of vitamins or related nutrients, non-limiting examples of which include vitamin A, vitamin E, vitamin D2, vitamin D3, vitamin A palmitate, vitamin E acetate, vitamin C palmitate (ascorbyl palmitate), vitamin K, thiamine, riboflavin, pyridoxine, vitamin B₁₂, carotenoids (*e.g*., beta-carotene, zeaxanthin, lutein, lycopene), niacin, folic acid, pantothenic acid, biotin, vitamin C, choline, inositol, salts and derivatives thereof, and combinations thereof. In yet other embodiments according to the first and second embodiments disclosed herein, the nutritional composition comprises any of a variety of additional minerals, non-limiting examples of which include calcium, selenium, potassium, iodine, phosphorus, magnesium, iron, zinc, manganese, copper, sodium, molybdenum, chromium, chloride, and combinations thereof.

In certain embodiments according to the first and second embodiments disclosed herein, the nutritional compositions optionally include one or more masking agents to reduce or otherwise obscure the development of any residual bitter flavors and after taste in the nutritional compositions over time. Suitable masking agents include natural and artificial sweeteners, sodium sources such as sodium chloride, and hydrocolloids, such as guar gum, xanthan gum, carrageenan, gellan gum, and combinations thereof. The amount of masking agent in certain embodiments of the nutritional composition may vary depending upon the particular masking agent selected, other ingredients in the formulation, and other formulation or product target variables. Such amounts, however, most typically range from 0.1% to 3%, including form 0.15% to 3%, and also including from 0.2% to 2.5%, by weight of the nutritional composition.

The various embodiments of the nutritional composition according to the first and second embodiments disclosed herein may be prepared by any process or suitable method (now known or known in the future) for making a selected product form, such as a nutritional solid, a nutritional powder, or a nutritional liquid. Many such techniques are known for any given product form such as nutritional liquids or nutritional powders and can easily be applied by one of ordinary skill in the art to the various embodiments of the nutritional composition according to the first and second embodiments disclosed herein.

In one suitable manufacturing process for liquid nutritional compositions according to the first and second embodiments disclosed herein, for example, at least three separate slurries are prepared, including a protein-in-fat (PIF) slurry, a carbohydrate-mineral (CHO-MIN) slurry, and a protein-in-water (PIW) slurry. The PIF slurry is formed by heating and mixing an oil (*e.g*., canola oil, corn oil, fish oil (including LCPUFAs), *etc.*) and then adding an emulsifier (*e.g*., lecithin), fat soluble vitamins, and a portion of the total protein (*e.g*., milk protein concentrate, etc.) with continued heat and agitation. The CHO-MIN slurry is formed by adding with heated agitation to water: minerals (*e.g*., potassium citrate, dipotassium phosphate, sodium citrate, *etc*.), trace and ultra trace minerals (TM/UTM premix), thickening or suspending agents (*e.g*., microcrystalline cellulose, gellan, carrageenan). The resulting CHO-MIN slurry is held for 10 minutes with continued heat and agitation before adding additional minerals *(e.g.,* potassium chloride, magnesium carbonate, potassium iodide, *etc.),* or carbohydrates *(e.g.,* prebiotic, sucrose, corn syrup, *etc.),* or combinations thereof. The PIW slurry is then formed by mixing with heat and agitation the remaining protein *(e.g.,* sodium caseinate, soy protein concentrate, *etc.)* into water.

In accordance with this process, the three resulting slurries are blended together with heated agitation and the pH adjusted to the desired range *(e.g.,* 6.6 to 7) after which the nutritional composition is subjected to high-temperature short-time (HTST) processing. The nutritional composition is heat treated, emulsified, homogenized, and cooled during HTST. Water soluble vitamins and ascorbic acid are added (if applicable), the pH is again adjusted (if necessary), flavors are added, and any additional water can be added to adjust the solids content to the desired range. At this point, the liquid nutritional composition may be packaged and sterilized according to any suitable sterilization technique, such as aseptic, retort, or hot-fill sterilization.

A nutritional solid suitable for use in the methods of the first and second embodiments disclosed herein, such as a spray dried nutritional powder or drymixed nutritional powder, may be prepared by any collection of known or otherwise effective technique suitable for making and formulating a nutritional powder. For example, when the nutritional powder is a spray dried nutritional powder, the spray drying step may likewise include any spray drying technique that is known for or otherwise suitable for use in the production of nutritional powders. Many different spray drying methods and techniques are known for use in the nutrition field, all of which are suitable for use in the manufacture of the spray dried nutritional powders herein.

One method of preparing the spray dried nutritional powder comprises forming and homogenizing an aqueous slurry or liquid comprising predigested fat, and optionally protein, carbohydrate, and other sources of fat, and then spray drying the slurry or liquid to produce a spray dried nutritional powder. The method may further comprise the step of spray drying, drymixing, or otherwise adding additional nutritional or functional ingredients, including any one or more of the ingredients described herein, to the spray dried nutritional powder.

As mentioned above, and in accordance with the first embodiment, an effective amount of LCPUFA is provided to an individual during pregnancy, during lactation, or during both pregnancy and lactation. Upon consumption of the effective amount of LCPUFA, the bone quality of the individual is maintained. In accordance with the second embodiment, an effective amount of a prebiotic is provided to an individual during pregnancy, during lactation, or during both pregnancy and lactation. Upon consumption of the effective amount of prebiotic, the bone quality of the individual is maintained. The terms "maintain," "maintained," or "maintenance" when used in connection with bone quality refers to retaining an amount of bone quality that corresponds to the bone quality of an individual prior to practicing the methods disclosed herein, or a percentage thereof, or an increase in bone quality. As previously noted, bone quality is indicated by various characteristics including, but not limited to, bone microarchitecture, bone mineral density (BMD), bone mineral content (BMC), bone morphology, accumulated microscopic damage, and bone turnover.

A variety of techniques may be used to determine the characteristics that provide an indication of bone quality. For example, a dual energy X-Ray absorptiometry (DEXA) scan may be used to measure BMC and BMD. BMC determines the mass of mineral present in the whole body or in a selected bone region. BMC changes reflect the result of the metabolic "mass" balance between bone formation and bone destruction. BMC represented relative to the projected bone area refers to BMD. BMD represents the whole mass of mineral present in the bone region studied. Other techniques for measuring BMC and BMD include single-photon absorptiometry, dual-photon absorptiometry, and quantitative computed tomography.

Another useful technique for providing an indication of bone quality is micro-computed tomography (micro-CT). A micro-CT scan allows for the nondestructive assessment and analysis of bone microarchitecture (e.g., three-dimensional trabecular and cortical bone structural properties). Scanning with micro-CT can be achieved at resolutions as low as 5 µm, allowing for the determination of porosities and subtle modeling and remodeling events of the bone tissue. Furthermore, true three-dimensional image reconstructions permit the assessment of bone microarchitecture as a three-dimensional structure, providing critical information to images collected through histomorphometry.

Bone turnover can be assessed by measurements of biochemical markers. Serum alkaline phosphatase, osteocalcin, and procollagen type I propeptides can be used as indices of bone formation, while urinary hydroxyproline and urinary pyridinoline and deoxypyridinoline can be used to assess bone resorption.

In certain embodiments according to the first embodiment, the bone quality that is maintained is bone microarchitecture. As mentioned above, bone microarchitecture may be assessed by a micro-CT scan to provide measurements of various parameters including, but not limited to, trabecular thickness, bone volume fraction (BV/TV), trabecular bone surface to bone volume ratio (BS/BV), structure model index (SMI), and connectivity density (Conn Dens). In certain embodiments according to the first embodiment, where the individual consumes the effective amount of LCPUFA during pregnancy, the level of maintenance at the end of pregnancy is from 60% to 100% of the bone microarchitecture of the individual prior to pregnancy including from 75% to 100%, including 80% to 100%, including from 90% to 100%, and also including from 95% to 100% of the bone microarchitecture of the individual prior to pregnancy. For determining the maintenance of bone microarchitecture, any one or more of the various parameters listed above *(e.g.,* trabecular thickness, BV/TV, BS/BV, *etc.)* may be utilized. The expression "prior to pregnancy" when used in connection with a measure of bone quality refers to a baseline measurement of bone quality that is measured prior to conception or after conception, but prior to practicing the methods disclosed herein.

In certain embodiments according to the first embodiment, the individual maintains bone microarchitecture during lactation (i.e., the period of time that the individual breastfeeds the offspring). For example, in certain embodiments according to the first embodiment where the individual consumes the effective amount of LCPUFA during lactation, the level of maintenance at the end of lactation is from 60% to 100% of the bone microarchitecture of the individual prior to lactation, including from 75% to 100%, including from 80% to 100%, including from 90% to 100%, and also including from 95% to 100% of the bone microacrhitecure of the individual prior to lactation. Again, the bone microarchitecture of the individual may be determined by various clinical techniques, and the bone microarchitecture of the individual may be determined prior to lactation (or less than two weeks after beginning to breastfeed the offspring), but prior to practicing the methods disclosed herein, and again after the lactation period is completed to assess the level of maintenance of bone microarchitecture.

In certain embodiments according to the first embodiment, the individual maintains bone microarchitecture during pregnancy and lactation. For example, in certain embodiments according to the first embodiment where the individual consumes the effective amount of LCPUFA during pregnancy and lactation, the level of maintenance at the end of lactation is from 60% to 100% of the bone microarchitecture of the individual prior to pregnancy, including from 75% to 100%, including from 80% to 100%, including from 90% to 100%, and also including from 95% to 100% of the bone microacrhitecure of the individual prior to pregnancy. Again, the bone microarchitecture of the individual may be determined by various clinical techniques, and the bone microarchitecture of the individual may be determined prior to conception or after conception, but prior to practicing the methods disclosed herein, and again after the lactation period is completed to assess the level of maintenance of bone microarchitecture.

In certain embodiments according to the first embodiment, the bone quality that is maintained is BMD. In certain embodiments according to the first embodiment, where the individual consumes the effective amount of LCPUFA during pregnancy, the level of maintenance at the end of pregnancy may be from 75% to 100% of the BMD of the individual prior to pregnancy, including from 80% to 100%, including from 90% to 100%, and also including from 95% to 100% of the BMD of the individual prior to pregnancy. As previously discussed, the BMD of the individual may be determined by various clinical techniques, such as DEXA, and may be determined prior to conception or after conception, but prior to practicing the methods disclosed herein, and again at the time of delivery or within a month of delivery to assess the level of BMD maintenance.

In certain embodiments according to the first embodiment, the individual maintains BMD during lactation. For example, in certain embodiments according to the first embodiment where the individual consumes the effective amount of LCPUFA during lactation, the level of maintenance at the end of lactation is from 75% to 100% of the BMD of the individual prior to lactation, including from from 80% to 100%, including from 90% to 100%, and also including from 95% to 100% of the BMD of the individual prior to lactation. Again, the BMD of the individual may be determined by various clinical techniques, and may be determined prior to lactation (or less than two weeks after beginning to breastfeed the offspring), but prior to practicing the methods disclosed herein, and again after the lactation period is completed to assess the level of maintenance of BMD.

In certain embodiments according to the first embodiment, the individual maintains BMD during pregnancy and lactation. For example, in certain embodiments according to the first embodiment where the individual consumes the effective amount of LCPUFA during pregnancy and lactation, the level of maintenance at the end of lactation may be from 75% to 100% of the BMD of the individual prior to pregnancy, including from 80% to 100%, including from 90% to 100%, and also including from 95% to 100% of the BMD of the individual prior to pregnancy. Again, the BMD of the individual may be determined by various clinical techniques, and the BMD of the individual may be determined prior to conception or after conception, but prior to practicing the methods disclosed herein, and again after the lactation period is completed to assess the level of BMD maintenance.

In certain embodiments according to the first embodiment, the bone quality that is maintained is BMC. In certain embodiments according to the first embodiment, where the individual consumes the effective amount of LCPUFA during pregnancy, the level of maintenance at the end of pregnancy may be from 75% to 100% of the BMC of the individual prior to pregnancy, including from 80% to 100%, including from 90% to 100%, and also including from 95% to 100% of the BMC of the individual prior to pregnancy. As previously discussed, the BMC of the individual may be determined by a number of clinical techniques, such as DEXA, and may be determined prior to conception or after conception, but prior to practicing the methods disclosed herein, and again at the time of delivery or within a month of delivery to assess the level of BMC maintenance.

In certain embodiments according to the first embodiment, the individual maintains BMC during lactation. For example, in certain embodiments according to the first embodiment where the individual consumes the effective amount of LCPUFA during lactation, the level of maintenance at the end of lactation is from 75% to 100% of the BMC of the individual prior to lactation, including from 80% to 100%, including from 90% to 100%, and also including from 95% to 100% of the BMC of the individual prior to lactation. As mentioned above, the BMC of the individual may be determined by various clinical techniques, and may be determined prior to lactation (or less than two weeks after beginning to breastfeed the offspring), but prior to practicing the methods disclosed herein, and again after the lactation period is completed to assess the level of maintenance of BMC.

In certain embodiments according to the first embodiment, the individual maintains BMC during pregnancy and lactation. For example, in certain embodiments according to the first embodiment where the individual consumes the effective amount of LCPUFA during pregnancy and lactation, the level of maintenance at the end of lactation may be from 75% to 100% of the BMC of the individual prior to pregnancy, including from 80% to 100%, including from 90% to 100%, and also including from 95% to 100% of the BMC of the individual prior to pregnancy. Again, the BMC of the individual may be determined by various clinical techniques, and the BMC of the individual may be determined prior to conception or after conception, but prior to practicing the methods disclosed herein, and again after the lactation period is completed to assess the level of BMC maintenance.

In certain embodiments according to the second embodiment, the bone quality that is maintained is bone microarchitecture. In certain embodiments according to the second embodiment, where the individual consumes the effective amount of prebiotic during pregnancy, the level of maintenance at the end of pregnancy may be from 60% to 100% of the bone microarchitecture of the individual prior to pregnancy, including from 75% to 100%, including from 80% to 100%, including from 90% to 100%, and also including from 95% to 100% of the bone microarchitecture of the individual prior to pregnancy. As mentioned above, the bone microarchitecture of the individual may be determined by various clinical techniques, such as micro-CT. Moreover, the bone microarchitecture may be determined prior to conception or after conception, but prior to practicing the methods disclosed herein, and again at the time of delivery or within a month of delivery to assess the level of maintenance of bone microarchitecture.

In certain embodiments according to the second embodiment, the individual maintains bone microarchitecture during lactation (i.e., the period of time that the individual breastfeeds the offspring). For example, in certain embodiments according to the second embodiment where the individual consumes the effective amount of prebiotic during lactation, the level of maintenance at the end of lactation is from 60% to 100% of the bone microarchitecture of the individual prior to lactation, including from 75% to 100%, including from 80% to 100%, including from 90% to 100%, and also including from 95% to 100% of the bone microacrhitecure of the individual prior to lactation. Again, the bone microarchitecture of the individual may be determined by various clinical techniques, and the bone microarchitecture of the individual may be determined prior to lactation (or less than two weeks after beginning to breastfeed the offspring), but prior to practicing the methods disclosed herein, and again after the lactation period is completed to assess the level of maintenance of bone microarchitecture.

In certain embodiments according to the second embodiment, the individual maintains bone microarchitecture during pregnancy and lactation. For example, in certain embodiments according to the second embodiment where the individual consumes the effective amount of prebiotic during pregnancy and lactation, the level of maintenance at the end of lactation is from 60% to 100% of the bone microarchitecture of the individual prior to pregnancy, including from 75% to 100%, including from 80% to 100%, including from 90% to 100%, and also including from 95% to 100% of the bone microacrhitecure of the individual prior to pregnancy. Again, the bone microarchitecture of the individual may be determined by various clinical techniques, and the bone microarchitecture of the individual may be determined prior to conception or after conception, but prior to practicing the methods disclosed herein, and again after the lactation period is completed to assess the level of maintenance of bone microarchitecture.

In certain embodiments according to the second embodiment, the bone quality that is maintained is BMD. In certain embodiments according to the second embodiment, where the individual consumes the effective amount of prebiotic during pregnancy, the level of maintenance at the end of pregnancy may be from 75% to 100% of the BMD of the individual prior to pregnancy, including from 80% to 100%, including from 90% to 100%, and also including from 95% to 100% of the BMD of the individual prior to pregnancy. As mentioned above, the BMD of the individual may be determined by various clinical techniques, such as DEXA. Moreover, the BMD may be determined prior to conception or after conception, but prior to practicing the methods disclosed herein, and again at the time of delivery or within a month of delivery to assess the level of BMD maintenance.

In certain embodiments according to the second embodiment, the individual maintains BMD during lactation. For example, in certain embodiments according to the second embodiment where the individual consumes the effective amount of prebiotic during lactation, the level of maintenance at the end of lactation is from 75% to 100% of the BMD of the individual prior to lactation, including from from 80% to 100%, including from 90% to 100%, and also including from 95% to 100% of the BMD of the individual prior to lactation. Again, the BMD of the individual may be determined by various clinical techniques, and may be determined prior to lactation (or less than two weeks after beginning to breastfeed the offspring), but prior to practicing the methods disclosed herein, and again after the lactation period is completed to assess the level of maintenance of BMD.

In certain embodiments according to the second embodiment, the individual maintains BMD during pregnancy and lactation. For example, in certain embodiments according to the first embodiment where the individual consumes the effective amount of prebiotic during pregnancy and lactation, the level of maintenance at the end of lactation may be from 75% to 100% of the BMD of the individual prior to pregnancy, including from 80% to 100%, including from 90% to 100%, and also including from 95% to 100% of the BMD of the individual prior to pregnancy. Again, the BMD of the individual may be determined by various clinical techniques, and the BMD of the individual may be determined prior to conception or after conception, but prior to practicing the methods disclosed herein, and again after the lactation period is completed to assess the level of BMD maintenance.

In certain embodiments according to the second embodiment, the bone quality that is maintained is BMC. In certain embodiments according to the second embodiment, where the individual consumes the effective amount of prebiotic during pregnancy, the level of maintenance at the end of pregnancy may be from 75% to 100% of the BMC of the individual prior to pregnancy, including from 80% to 100%, including from 90% to 100%, and also including from 95% to 100% of the BMC of the individual prior to pregnancy. As previously discussed, the BMC of the individual may be determined by a number of clinical techniques, such as DEXA, and may be determined prior to conception or after conception, but prior to practicing the methods disclosed herein, and again at the time of delivery or within a month of delivery to assess the level of BMC maintenance.

In certain embodiments according to the second embodiment, the individual maintains BMC during lactation. For example, in certain embodiments according to the second embodiment where the individual consumes the effective amount of prebiotic during lactation, the level of maintenance at the end of lactation is from 75% to 100% of the BMC of the individual prior to lactation, including from 80% to 100%, including from 90% to 100%, and also including from 95% to 100% of the BMC of the individual prior to lactation. As mentioned above, the BMC of the individual may be determined by various clinical techniques, and may be determined prior to lactation (or less than two weeks after beginning to breastfeed the offspring), but prior to practicing the methods disclosed herein, and again after the lactation period is completed to assess the level of maintenance of BMC.

In certain embodiments according to the second embodiment, the individual maintains BMC during pregnancy and lactation. For example, in certain embodiments according to the second embodiment where the individual consumes the effective amount of prebiotic during pregnancy and lactation, the level of maintenance at the end of lactation may be from 75% to 100% of the BMC of the individual prior to pregnancy, including from 80% to 100%, including from 90% to 100%, and also including from 95% to 100% of the BMC of the individual prior to pregnancy. Again, the BMC of the individual may be determined by various clinical techniques, and the BMC of the individual may be determined prior to conception or after conception, but prior to practicing the methods disclosed herein, and again after the lactation period is completed to assess the level of BMC maintenance.
Accordingly, supplementation with an effective amount of LCPUFA, or an effective amount of prebiotic, or both an effective amount of LCPUFA and an effective amount of prebiotic can provide a level of bone protection (i.e., maintenance of bone quality) to a pregnant or lactating individual. While not wishing to be bound by any particular theory, it is believed that consumption of an effective amount of a LCPUFA, an effective amount of a prebiotic, or both will result in an increase intestinal calcium absorption, promote osteoblastogenesis, and decrease osteoclastogenesis and osteoclast activity.

### EXAMPLES

The following examples illustrate certain embodiments or features of the methods according to the first and second embodiments disclosed herein. The examples are given solely for the purpose of illustration and are not to be construed as limitations of the present disclosure, as many variations thereof are possible without departing from the spirit and scope of the disclosure.

### Example 1

Examples 1 illustrates certain embodiments according to the first embodiment disclosed herein of a method for maintaining bone quality in a pregnant or lactating individual.

Twenty 10-week-old pregnant Sprague-Dawley rats between the tenth to twelfth day of gestation, and ten 10-week-old non-pregnant, non-lactating (NP/NL) female Sprague-Dawley rats (Charles Rivers Laboratories, Orleans Cedex, France) were housed under standardized environmental conditions (22° C, relative humidity of 50%, artificial lighting on for 12 hours/day) and were given free access to deionized water during the test period. The pregnant rats were randomly divided into two groups of feeding (n=10/group): (1) Control Group ("Control"), who received a standard purified rodent diet (SPRD) fortified with 0.5% calcium carbonate; and (2) LCPUFA group ("LCPUFA"), who receives the SPRD containing 50% of the total fat in the form of n-3 long chain polyunsaturated fatty acids (Eupoly-DHA™, Puleva Biotech, Spain). The non-pregnant, non-lactating rats received the SPRD.

After delivery, pups from the same feeding group were mixed and 8 pups were randomly housed to one dam from the same feeding group. During their suckling period, the pups received only the dam's milk. The dam's nutritional treatment finished at the end of the lactation period (pups, 28-day-old).

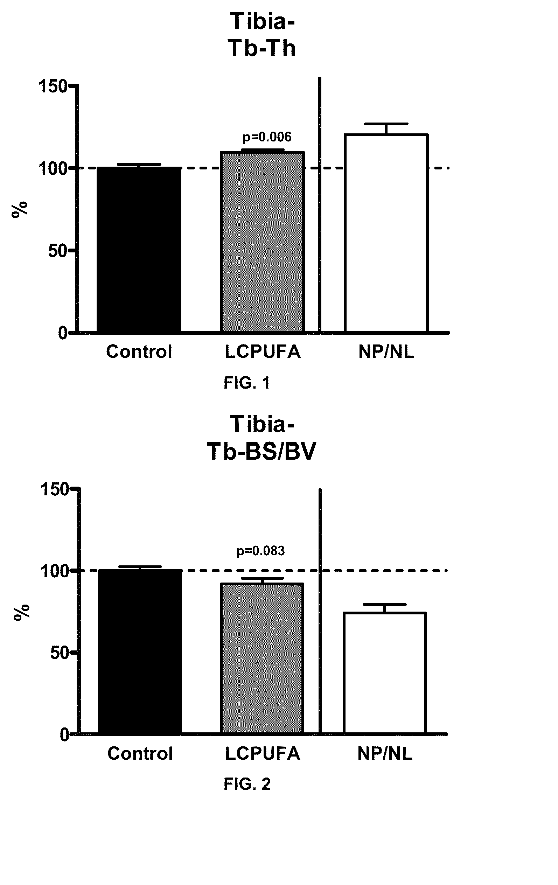

Sacrifices of rat dams and NP/NL rats were carried out at the end of the lactation period. Determination of trabecular architecture parameters: trabecular thickness (Tb-Th), trabecular bone surface to bone volume ratio (BS/BV), and Structure Model Index (SMI) were analyzed by micro-computed tomography (micro-CT). Micro-CT analysis was performed by SCANCO Medical AG (Brüttisellen, Switzerland). Data for the bone measurements is provided in Table 1 below, with Figures 1-3 showing a graphical representation of certain data.

**Table 1**

| | Control | LCPUFA | NP/NL |
|---|---|---|---|
| Tb.Th (%) | 100.0 ± 2.2 | 109.5 ± 1.7¹ | 120.4 ± 6.4 |
| SMI (%) | 100.0 ± 2.4 | 91.9 ± 3.8² | 18.7 ± 18.5 |
| BS/BV (%) | 100.0 ± 2.6 | 92.0± 3.4² | 74.5 ± 5.1 |

| | | | |
|---|---|---|---|
| ¹ p-value< 0.05 ² p-value< 0.1 | | | |

As can be seen from the data, at the time of sacrifice (*i.e*., at the end of the lactation period), the dams who received the LCPUFA supplemented diet showed an improvement in their trabecular architecture (as determined by micro-CT analysis). For example, the tibia trabecular thickness (Tibia Tb-Th) of the LCPUFA group showed a significant improvement (p<0.05) with respect to the Control group, as seen in FIG. 1. In other words, even though the dams that received LCPUFA showed a decrease in tibia trabecular thickness as compared to the NP/NL rats, the amount of decrease was less in those rats experiencing pregnancy and lactation that received the LCPUFA as compared to the rats experiencing pregnancy and lactation that did not receive the LCPUFA.

As seen in FIG. 2, the tibia trabecular bone surface to bone volume ratio (Tibia Tb-BS/BV) of the LCPUFA group improved with respect to the Control group. The BS/BV parameter provides an indication of the proportion of bone surface that is available for remodeling. Therefore, a higher BS/BV indicates a higher rate of bone turnover, which can reduce bone strength. As FIG. 2 demonstrates, the dams that received LCPUFA showed a higher tibia Tb-BS/BV as compared to the NP/NL rats, but showed a lower tibia Tb-BS/BV as compared to the dams that did not receive the LCPUFA (*i.e.,* Control group).

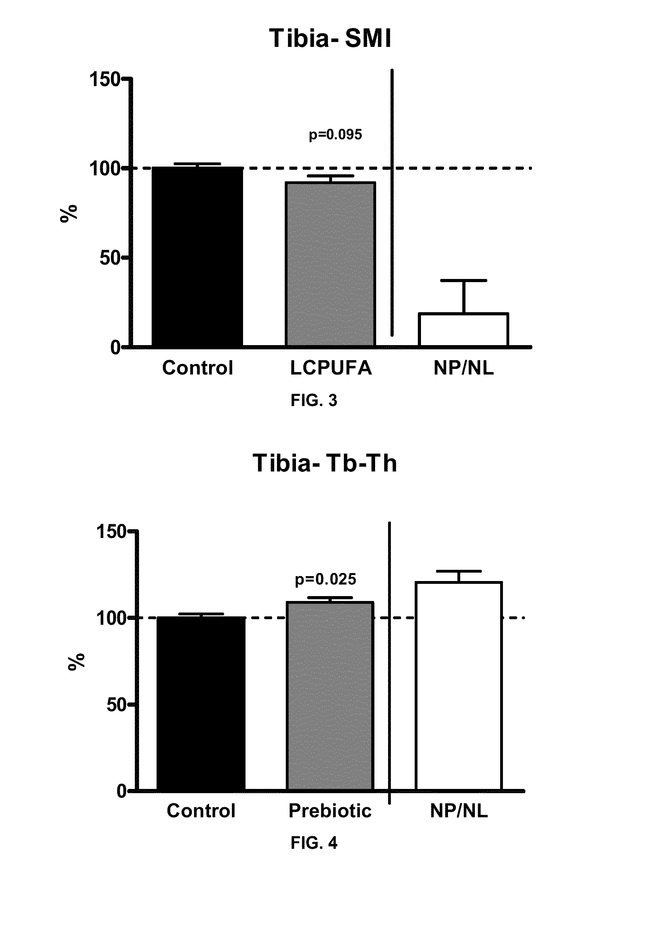

Moreover, the Structure Model Index (SMI) of the LCPUFA group, as illustrated in FIG. 3, showed an improvement compared to the SMI of the Control group. The SMI is a parameter that provides an indication of bone structure in terms of "rod-like" and "plate-like" structures. For an ideal plate and rod structure the SMI value is 0 and 3, respectively. Bone having a more plate-like structure, and thus a lower SMI, is considered to be stronger. Thus, as FIG. 3 demonstrates, the NP/NL rats had a much lower SMI compared to the LCPUFA group and the Control group, but dams that received LCPUFA showed a lower SMI compared to the Control group dams that did not receive the LCPUFA.

### Example 2

Examples 2 illustrates certain embodiments according to the second embodiment disclosed herein of a method for maintaining bone quality in a pregnant or lactating individual.

Twenty 10-week-old pregnant Sprague-Dawley rats between the tenth to twelfth day of gestation, and ten 10-week-old non-pregnant, non-lactating (NP/NL) female Sprague-Dawley rats (Charles Rivers Laboratories, Orleans Cedex, France) were housed under standardized environmental conditions (22° C, relative humidity of 50%, artificial lighting on for 12 hours/day) and were given free access to deionized water during the test period. The pregnant rats were randomly divided into two groups of feeding (n=10/group): (1) Control Group ("Control"), who received a standard purified rodent diet (SPRD) with 0.5% calcium carbonate; and (2) Prebiotic group ("Prebiotic"), who received the SPRD containing 7.5% of the total carbohydrate as inulin-type fructans (Synergy-1™, Orafti, Belgium). The NP/NL rats received the SPRD.

After delivery, pups from the same feeding group were mixed and 8 pups were randomly housed to one dam from the same feeding group. During their suckling period, the pups received only the dam's milk. The dam's nutritional treatment was finished at the end of the lactation period (pups, 28-day-old).

Sacrifices of rat dams and NP/NL rats were carried out at the end of the lactation period. Determination of trabecular architecture parameters: trabecular thickness (Tb-Th), trabecular bone surface to bone volume ratio (BS/BV), Structure Model Index (SMI), bone volume to total volume ratio (BV/TV), and Connectivity Density (Conn-Dens) were analyzed by micro-computed tomography (micro-CT). Micro-CT analysis was carried out by SCANCO Medical AG (Brüttisellen, Switzerland). Data for the bone measurements is provided in Table 2 below, with Figures 4-8 showing a graphical representation of certain data.

**Table 2**

| | Control | LCPUFA | NP/NL |
|---|---|---|---|
| BV/TV (%) | 100.0 ± 7.3 | 135.3± 16.7² | 263.5 ± 28.8 |
| Tb.Th (%) | 100.0 ± 2.2 | 109.0 ± 2.8¹ | 120.4 ± 6.4 |
| Conn Dens (%) | 100.0 ± 6.2 | 135.7 ± 19.8² | 276.4 ± 20.0 |
| SMI (%) | 100.0 ± 2.4 | 81.1 ± 5.5¹ | 18.7 ± 18.5 |
| BS/BV (%) | 100.0 ± 2.6 | 88.0 ± 3.0¹ | 74.5 ± 5.1 |

| | | | |
|---|---|---|---|
| ¹ p-value< 0.05 ² p-value< 0.1 | | | |

As can be seen from the data, at the time of sacrifice (*i.e*., at the end of the lactation period), the dams who received the Prebiotic supplemented diet showed an improvement in their trabecular architecture (as determined by micro-CT analysis). For example, the tibia trabecular thickness (Tibia Tb-Th) of the Prebiotic group showed a statistically significant improvement with respect to the Control group, as seen in FIG. 4. In other words, even though the dams that received Prebiotic showed a decrease in tibia trabecular thickness as compared to the NP/NL rats, the amount of decrease was less in the dams that received the Prebiotic as compared to the dams that did not receive the Prebiotic (*i.e.,* the Control group).

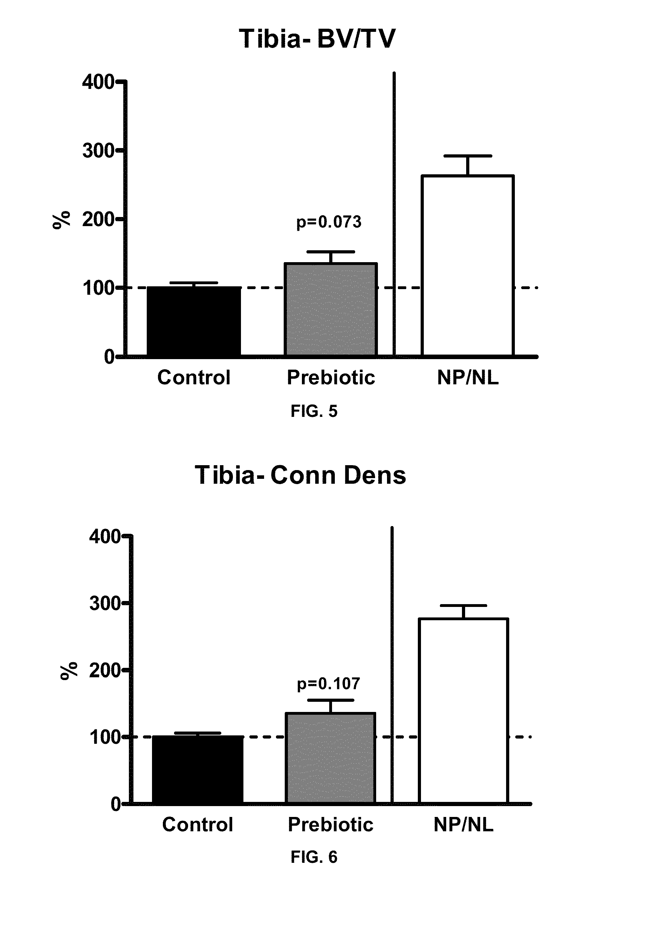

As seen in FIG. 5, the tibia bone volume to total volume ratio (Tibia BV/TV) of the Prebiotic group showed an improvement with respect to the Control group. The BV/TV parameter provides an indication of the fraction of total bone volume that is occupied by mineralized bone. Therefore, a comparatively higher BV/TV is an indicator of increased bone density. As FIG. 5 demonstrates, the dams that received Prebiotic showed a lower tibia BV/TV as compared to the NP/NL rats, but showed a higher tibia BV/TV as compared to the dams that did not receive the Prebiotic (i.e., Control group).

The Connectivity Density (Conn Dens) is another parameter that provides an indication of trabecular bone architecture. The Conn Dens is a measure of the degree of connectivity of the trabeculae within a given volume. A higher Conn Dens is generally an indication of better bone quality. As seen in FIG. 6, the tibia Conn Dens of the Prebiotic group was lower than the tibia Conn Dens of the NP/NL rats. On the other hand, the dams that received Prebiotic showed a higher tibia Conn Dens relative to the dams of the Control group, which did not receive Prebiotic.

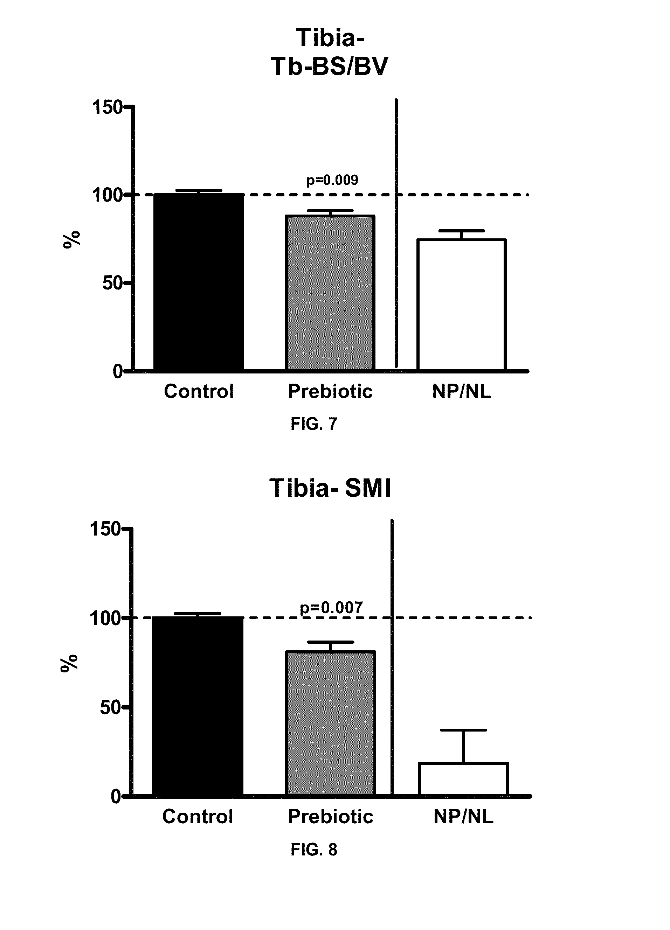

As seen in FIG. 7, the tibia trabecular bone surface to bone volume ratio (Tibia Tb-BS/BV) of the Prebiotic group improved relative to the Control group. The BS/BV parameter provides an indication of the proportion of bone surface that is available for remodeling. Therefore, a higher BS/BV indicates a higher rate of bone turnover, which can reduce bone strength. As FIG. 7 demonstrates, the dams that received Prebiotic showed a higher tibia Tb-BS/BV as compared to the NP/NL rats, but showed a lower tibia Tb-BS/BV as compared to the dams of the Control group, which did not receive Prebiotic.

In addition, the Structure Model Index (SMI) of the Prebiotic group showed an improvement compared to the SMI of the Control group, as illustrated in FIG. 8. The SMI is a parameter that provides an indication of bone structure in terms of "rod-like" and "plate-like" structures. For an ideal plate and rod structure the SMI value is 0 and 3, respectively. Bone having a more plate-like structure, and thus a lower SMI, is considered to be stronger. Thus, as FIG. 8 demonstrates, the NP/NL rats had a much lower SMI compared to the Prebiotic group and the Control group, but dams that received Prebiotic showed a lower SMI relative to the Control group dams that did not receive the Prebiotic.

### Examples 3-5

Examples 3-5 illustrate certain embodiments according to the first and second embodiments disclosed herein of a nutritional composition in the form of a nutritional powder. The nutritional powders may be prepared by spray drying methods or dry blending, and may be reconstituted with water prior to use to the desired target ingredient concentrations. All ingredient amounts are listed as kilogram per 1000 kilogram batch of product, unless otherwise specified.

**Table 3**

| **Ingredient** | **Ex. 3** | **Ex. 4** | **Ex. 5** |
|---|---|---|---|
| Skim Milk Powder | 655.5 | 582.0 | 519.65 |
| Short Chain FOS | 100.0 | 150.0 | 200.0 |
| Extra Fine White Sugar | 81.1 | 81.1 | 81.1 |
| Whole Milk Powder | 44.8 | 44.8 | 44.8 |
| Calcium Phosphate Dibasic | 24.1 | 24.1 | 24.1 |
| Magnesium Phosphate Dibasic | 19.1 | 19.1 | 19.1 |
| Choline Premix | 10.3 | 10.3 | 10.3 |
| Vitamin/Mineral Premix | 8.0 | 8.0 | 8.0 |
| Flavor | 6.0 | 6.0 | 6.0 |
| Powdered DHA (11% (w/w) DHA) | 47.0 | 70.5 | 82.85 |
| Sodium Ascorbate | 3.78 | 3.78 | 3.78 |
| Milk Flavor Powder | 1.5 | 1.5 | 1.5 |

To the extent that the term "includes" or "including" is used in the specification or the claims, it is intended to be inclusive in a manner similar to the term "comprising" as that term is interpreted when employed as a transitional word in a claim. Furthermore, to the extent that the term "or" is employed (e.g., A or B) it is intended to mean "A or B or both." When the applicants intend to indicate "only A or B but not both" then the term "only A or B but not both" will be employed. Thus, use of the term "or" herein is the inclusive, and not the exclusive use. See Bryan A. Garner, A Dictionary of Modern Legal Usage 624 (2d. Ed. 1995). Also, to the extent that the terms "in" or "into" are used in the specification or the claims, it is intended to additionally mean "on" or "onto." Furthermore, to the extent the term "connect" is used in the specification or claims, it is intended to mean not only "directly connected to," but also "indirectly connected to" such as connected through another component or components.

While the present application has been illustrated by the description of embodiments thereof, and while the embodiments have been described in considerable detail, it is not the intention of the applicants to restrict or in any way limit the scope of the appended claims to such detail. Additional advantages and modifications will readily appear to those skilled in the art. Therefore, the application, in its broader aspects, is not limited to the specific details, the representative compositions and processes, and illustrative examples shown and described. Accordingly, departures may be made from such details without departing from the spirit or scope of the applicant's general inventive concept.

## Claims

1. A method for maintaining bone quality in an individual during pregnancy, during lactation or during pregnancy and lactation comprising:
providing an effective amount of a long chain polyunsaturated fatty acid for oral consumption by the individual during pregnancy, during lactation, or during pregnancy and lactation;
whereby the bone quality of the individual is maintained upon consumption of the effective amount of the long chain polyunsaturated fatty acid.

2. The method according to claim 1, wherein the long chain polyunsaturated fatty acid is selected from the group consisting of n-3 fatty acids, n-6 fatty acids, and combinations thereof.

3. The method according to claim 1, wherein the bone quality that is maintained is bone microarchitecture and the level of maintenance at the end of the period of consumption is 60-100% of the bone micorarchitecture of the individual prior to pregnancy.

4. The method according to claim 1, wherein the long chain polyunsaturated fatty acid is provided as part of a nutritional composition.

5. The method according to claim 4, wherein the nutritional composition further comprises an effective amount of a prebiotic.

6. The method according to any one of claims 1-5, wherein the long chain polyunsaturated fatty acid is selected from the group consisting of docosahexaenoic acid, eicosapentaenoic acid, α-linolenic acid, arachidonic acid, linoleic acid, γ-linolenic acid, and combinations thereof.

7. The method according to claim 4, wherein the nutritional composition contains 150-500 kcal per serving and is in the form of a liquid, a powder suitable for reconstitution to a liquid, or a bar.

8. The method according to claims 4, 5 or 7, wherein the nutritional composition further comprises at least one source of carbohydrate and at least one source of protein.

9. The method according to any one of claims 1-5 or 7, wherein the effective amount of long chain polyunsaturated fatty acid is consumed daily during pregnancy.

10. The method according to any one of claims 1-5 or 7, wherein the effective amount of long chain polyunsaturated fatty acid is consumed daily during pregnancy and lactation.

11. The method according to any one of claims 1-5 or 7, wherein the effective amount of long chain polyunsaturated fatty acid ranges from 100 mg/day to 3000 mg/day.

12. A method for maintaining bone quality in an individual during pregnancy, during lactation, or during pregnancy and lactation comprising:
providing an effective amount of a prebiotic for oral consumption by an individual during pregnancy, during lactation or during pregnancy and lactation;
whereby the bone quality of the individual is maintained upon consumption of the effective amount of the prebiotic.

13. The method according to claim 12, wherein the prebiotic is selected from the group consisting of inulin, short and long chain fructooligosaccharides, galactooligosaccharides, xylooligosaccharides, galactofructose, resistant starches, and combinations thereof.

14. The method according to claim 12, wherein the bone quality that is maintained is bone microarchitecture and the level of maintenance at the end of the period of consumption is 60-100% of the bone microarchitecture of the individual prior to pregnancy.

15. The method according to claim 12, wherein the prebiotic is provided as part of a nutritional composition.

16. The method according to claim 15, wherein the nutritional composition further comprises a long chain polyunsaturated fatty acid.

17. The method according to claim 15 or 16, wherein the nutritional composition contains 150-500 kcal per serving and is in the form of a liquid, a powder suitable for reconstitution to a liquid, or a bar.

18. The method according to claim 15 or 16, wherein the nutritional composition comprises at least one source of fat, at least one source of carbohydrate, and at least one source of protein.

19. The method according to any one of claims 12-16, wherein the prebiotic is consumed daily during pregnancy.

20. The method according to any one of claims 12-16, wherein the effective amount of prebiotic ranges from 0.001 g/day to 15 g/day.

21. Use of long chain polyunsaturated fatty acids for the maintenance of bone quality in an individual during pregnancy, during lactation, or during pregnancy and lactation.

22. Use of prebiotics for the maintenance of bone quality in an individual during pregnancy, during lactation, or during pregnancy and lactation.
